# EUROPÄISCHE PATENTANMELDUNG

(11) **EP 0 814 072 A2**
(43) Veröffentlichungstag der Anmeldung: **29.12.1997**
(21) Anmeldenummer: 97109283.8
(22) Anmeldetag: 09.06.1997
(51) Int. Cl.: C07C 37/08, C07C 37/82, C07C 39/16

(54) **Verfahren zur Thermostabilisierung von Bisphenolen**

(30) Priorität: 21.06.1996 DE 19624765; 09.10.1996 DE 19641527
(71) Anmelder: BAYER AG, 51368 Leverkusen (DE)
(72) Erfinder: Heydenreich, Frieder, Dr., 40593 Düsseldorf (DE); Wulff, Claus, Dr., 47800 Krefeld (DE); Fennhoff, Gerhard, Dr., (BE); Lanze, Rolf, Dipl.Ing., 47804 Krefeld (DE); Neumann, Rainer, Dr., 47803 Krefeld (DE)

(57) **Zusammenfassung**

Gegenstand der Erfindung ist ein Verfahren zur Herstellung thermostabiler Bisphenole, bei dem
a) in im Prinzip bekannter Weise ein Phenol und ein Keton oder Aldehyd katalytisch an einem sauren Ionenaustauscherharz in Gegenwart eines Cokatalysators zu dem entsprechenden Bisphenol umgesetzt werden,
b) das nach a) erzeugte Reaktionsgemisch durch einen Behälter, welcher mit einem anorganischen Oxid gefüllt ist, geleitet wird,
c) aus dem nach b) gewonnenen Reaktionsgemisch in im Prinzip bekannter Weise Bisphenol isoliert wird, den üblichen Reinigungsschritten unterzogen und gegebenenfalls das in einem weiteren Verfahrensschritt zunächst erstarrte Bisphenol zur Weiterverarbeitung aufgeschmolzen wird.

## Beschreibung

Bisphenol-Phenoladdukte und Bisphenole, die nach den üblichen in der Literatur bekannten Verfahren durch Katalyse an einem sauren Ionenaustauscherharz hergestellt und nach dem Fachmann bekannten Verfahren weiter aufgearbeitet werden, unterliegen während der thermischen Aufarbeitung Zersetzungsreaktionen. Folge dieser Zersetzungen sind die Verschlechterung der Reinheit und Farbqualität der Bisphenole. Dies wirkt sich auch auf die Qualität der aus den Bisphenolen hergestellten Produkte wie Epoxidharze, Polyester, Polyestercarbonate und Polycarbonate negativ aus, und zwar dergestalt, daß Farbprobleme, schlechte Lichtdurchlässigkeit transparenter Produkte oder Stippen in den Oberflächen von Formteilen aus diesen Endprodukten die Folge sind. Deshalb ist es von besonderem Interesse, die thermische Stabilität der Bisphenol-Phenoladdukte und Bisphenole zu verbessern.

Verfahren zur Stabilisierung von Bisphenolen sind in der Literatur bereits beschrieben, weisen jedoch erhebliche Nachteile auf:

Gemäß EP-A 374 692 werden Hydroxycarbonsäuren bzw. auch deren Ammonium- und Alkalimetallsalze als Additive eingesetzt. Diese Zusatzstoffe veninreinigen jedoch das Bisphenol selbst und können in den Nachfolgeprodukten störend wirken und dort zu erheblichen Qualitätseinbußen führen.

In der EP-A 523 931 werden dem jeweiligen Bisphenol-Phenoladdukt Alkalisalze aliphatischer Carbonsäuren zugesetzt. Diesem Verfahren müssen allerdings die gleichen Nachteile zugeschrieben werden, wie dem in der EP-A 374 692 beschriebenen Verfahren.

In der US-PS 5 399 789 werden den jeweiligen Bisphenolen vor der thermischen Aufarbeitung Amine in Mengen zwischen 1 bis 1 000 ppm zugesetzt. Hier sind einerseits die bereits für die EP-A 374 692 benannten Nachteile zu berücksichtigen, andererseits führen Überdosierungen von basischen Substanzen wie z.B. Aminen zu Produktzersetzungen in thermischen Auf- und Weiterarbeitungsprozessen.

EP-A 469 689 beschreibt ein Verfahren, in dem die adsorbtive Wirkung von Aktivkohle genutzt wird, um das Bisphenol vor Spaltprozessen in den nachfolgenden Aufarbeitungsstufen zu bewahren. Hohe Reaktionsgemischdurchsätze, insbesondere in kontinuierlicher Betriebsweise, führen jedoch selbst bei Verwenden von Aktivkohlegranulaten zu Feinstabrieb der Aktivkohle, der immer wieder, auch bei sorgfältigster Filtration, bis ins Endprodukt gelangen kann und so in Form schwarzer Partikel die farbliche Qualität und allgemeine Reinheit der Produkte sehr verschlechtert.

In EP-A 559 372 wird die Kombination eines sauren und basischen Ionenaustauscherharzes in Form eines Mischbettes beschrieben, durch welches Mutterlaugen geleitet und so gereinigt werden, bevor sie dem Eduktgemisch vor der eigentlichen Reaktion zugesetzt werden. Dieses Verfahren hat jedoch den Nachteil, daß zersetzend wirkende Substanzen, die erst im Verlaufe der Reaktion in das Produktgemisch gelangen, nicht entfernt werden und so weiterhin die Produktqualität verschlechtern können. Zudem gelangen stets Oligomere dieser Harze in gelöster Form in den Produktstrom und verschlechtern ebenfalls die Produktqualität.

Es ist nun gelungen, die Thermostatilität von Bisphenol-Phenoladdukten und Bisphenolen durch einen einfachen, im großtechnischen Maßstab leicht umsetzbaren Verfahrensschritt deutlich zu verbessern.

Gegenstand der Erfindung ist ein Verfahren zur Herstellung thermostabiler Bisphenole, bei dem
a) in im Prinzip bekannter Weise ein Phenol und ein Keton oder Aldehyd katalytisch an einem sauren Ionentauscherharz in Gegenwart eines Cokatalysators zu dem entsprechenden Bisphenol umgesetzt werden
b) das nach a) erzeugte Reaktionsgemisch durch einen Behälter, welcher mit einem anorganischen Oxid gefüllt ist, geleitet wird,
c) aus dem nach b) gewonnenen Reaktionsgemisch in im Prinzip bekannter Weise Bisphenol isoliert wird.

In der Regel fällt das durch Katalyse an einem sauren Ionenaustauscherharz hergestellte Bisphenol in einem Gemisch an, das Nebenprodukte, das als Edukt verwendete Phenol, Mutterlauge, die nach Abtrennen der Bisphenol-Phenoladdukte von der Lösung anfällt, Reste der als Edukt eingesetzten Carbonylkomponente, Cokatalysatoren wie Mercaptane und Reaktionswasser enthalten kann. Dieses Reaktionsgemisch wird in flüssiger Form in oder durch eine Vorlage, ein Rohr oder einen sonstigen geeigneten Behälter geleitet, der mit einem anorganischen Oxid teilweise oder vollständig gefüllt ist. Dabei ist es für die nachfolgenden Schritte und die zu erzielende thermische Stabilität der Produkte unerheblich, ob das vorgenannte anorganische Oxid in Form eines Festbettes, Wirbelbettes, Fließbettes oder in einem Rührkessel mit dem Reaktionsgemisch in Kontakt gebracht werden und die Fließrichtung durch das jeweilige Gefäß von unten nach oben oder von oben nach unten gerichtet ist. Auch ist es für die Verbesserung der Endproduktqualität nicht von Bedeutung, ob das vorgenannte anorganische Oxid als Pulver, feinkörnig, grobkörnig oder in Granulatform vorliegt, besonders bevorzugt sind jedoch grobkörnige anorganische Oxide und Granulate.

Anschließend werden die so behandelten Reaktionsmischungen den üblichen, dem Fachmann bekannten Reinigungs- und Aufarbeitungsschritten wie Kristallisation, Filtration oder Zentrifugieren unterzogen, wobei die resultierende Mutterlauge wieder für den Syntheseschritt verwendet werden kann. Die Abtrennung des Phenols vom Bisphenol kann z.B. durch Destillation oder Extraktion erfolgen. Dabei zeigt sich überraschenderweise, daß die so behandelten Produkte ohne jegliche Qualitätseinbußen die thermischen Aufarbeitungsstufen wie beispielsweise das destillative Abtrennen der Bisphenole von Phenolen, die destillative Reinigung der Bisphenole von den übrigen Begleit- und Nebenprodukten, aber auch spätere thermische Belastungen z.B. in Form von Aufschmelzprozessen, wie sie oftmals für eine Weiterverarbeitung notwendig sind, überstehen.

Ferner zeigt sich bei Bisphenol A überraschenderweise ein weiterer positiver Effekt: Im Produkt vorhandenes o,p'-Bisphenol A wird nach Behandlung der phenolischen Lösungen mit den vorgenannten anorganischen Oxiden während der thermischen Behandlung teilweise zu p,p'-Bisphenol A isomerisiert. Auf diese Weise wird der Anteil des unerwünschten o,p'-Isomeren im Bisphenol A zugunsten des erwünschten p,p'-Isomeren reduziert, wodurch die Produktreinheit insgesamt zunimmt.

Ein weiterer überraschender Effekt ist die deutliche Farbaufhellung der phenolischen Produktlösungen nach der Behandlung mit anorganischen Oxiden. Diese Verbesserung der farblichen Qualität des Rohproduktes, gemessen als Iodfarbzahl, äußert sich auch und vor allem in einer Verbesserung der Farbqualität des Endproduktes, d.h. die Farbe des reinen Bisphenols in der Schmelze nach dem Abtrennen des Phenols, gemessen als Hazen Farbzahl, wird besser.

Anders als in EP-A 374 692 und US-PS 5 399 789 werden im erfindungsgemäßen Verfahren die Produkte nicht durch Zusatzstoffe verunreinigt, da die oben beschriebenen Aluminiumoxide nicht im Produkt verbleiben, sondern durch entsprechende zwischengeschaltete Abtrennaggregate wie Kies- und/oder Sandbetten und/oder herkömmliche Filter von dem jeweiligen Reaktionsgemisch abgetrennt werden können.

Die eingesetzten anorganischen Oxide weisen hohe Abriebfestigkeiten auf, so daß keine hohen Festpartikelanteile gebildet werden, die in das Endprodukt gelangen und dessen Qualität beeinträchtigen könnten. Die anorganischen Oxide können sogar selbst als mechanisches Filter wirken und Feststoffpartikel, z.B. aus dem Katalysatorbett herausgelöste Teilchen, aus dem Produkt entfernen.

Anorganische Oxide im Sinne der Erfindung sind Magnesiumoxide, Calciumoxide, Bariumoxide, Titanoxide und Zinkoxide, bevorzugt sind Siliziumoxide und Alumosilikate (insbesondere Tonerden und Zeolithe), besonders bevorzugt ist Al₂O₃. Die vorgenannten anorganischen Oxide können sowohl in reiner Form als auch als Gemisch mit anderen Oxiden eingesetzt werden, wobei auch Oxide der Alkalimetalle zugegen sein können. In solchen Fällen sind besonders bevorzugt Oxide, die Anteile von 0,1 Gew.-% bis 1 Gew.-% Natriumoxid und/oder Kaliumoxid enthalten.

Beispiele für nach dem erfindungsgemäßen Verfahren herstellbare Bisphenole sind Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfoxide, Bis-(hydroxyphenyl)-sulfone, α,α'-Bis-(hydroxyphenyl)-diisopropylbenzole.
Bevorzugt sind 4,4'-Dihydroxydiphenyl, 2,2-Bis-(4-hydroxyphenyl)-propan, 2,4-Bis-(4-hydroxyphenyl)-2-methylbutan, 1,1-Bis-(4-hydroxyphenyl)-cyclohexan, α,α'-Bis-(4-hydroxyphenyl)-p-diisopropylbenzol, 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-hydroxyphenyl)-methan, Bis-(3,5-dimethyl-4-hydroxyphenyl)-sulfon, 2,4-Bis-(3,5-dimethyl-4-hydroxyphenyl)-2-methylbutan, 1,1-Bis-(3,5-dimethyl-4-hydroxyphenyl)-cyclohexan, 1,1-Bis-(4-hydroxyphenyl)-3,3,5-trimethylcyclohexan, α,α'-Bis-(3,5-dimethyl4-hydroxyphenyl)-p-diisopropylbenzol und 2,2-Bis-(3,5-dichlor-4-hydroxyphenyl)-propan;
besonders bevorzugt 2,2-Bis-(4-hydroxyphenyl)-propan, 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan und 1,1-Bis-(4-hydroxyphenyl)-3,3,5-trimethylcyclohexan.

### Beispiele

### A. Anorganische Oxide im Rührversuch

### A.1) Herstellung der Testlösungen

Eine Glassäule (80 mm Innendurchmesser) mit Außenmantelbeheizung (65°C) wurde zunächst mit Phenol gefüllt, dann mit Sand (Füllhöhe 50 mm) und zuletzt mit 300 g eines phenolfeuchten, sauren Ionenaustauscherharzes (sulfoniertes Polystyrolharz, vernetzt mit 4 Gew.-% Divinylbenzol, Typ K 1221, Bayer AG) beschickt. Das Ionenaustauscherharz war zuvor mit entsalztem Wasser bei 60 bis 80°C im Durchfluß auf Leitfähigkeiten des wäßrigen Eluates < 5 µS/cm⁻¹ gewaschen und anschließend mit 70°C warmen Phenol entwässert worden, bis der Wassergehalt des Phenols unter 0,15 Gew.-% lag.

Durch diese Säule wurde bei 65°C ein Gemisch aus 96 Gew.-% Phenol, 4 Gew.-% Aceton und 150 ppm Mercaptopropionsäure mit einem Durchsatz von 100 g/h gepumpt. Nach vierwöchiger Säulenlaufzeit wurde am Säulenaustritt eine Probe von 600 ml Reaktionsgemisch entnommen und in zwei Proben zu je 300 ml geteilt.

### A.2) Behandlung der Teillösungen

Die eine Probenhälfte wurde bei 80°C mit 10 g Aluminiumoxid (1 mm bis 2,5 mm Granulatdurchmesser,Typ AN/V-812, Martinswerk GmbH, D-50127 Bergheim) 3 Stunden lang verrührt. Anschließend wurde das Aluminiumoxid abfiltriert, das Phenol bei 120°C und 100 mm abdestilliert und der Rückstand bei 180°C 30 Minuten lang getempert. Gaschromatographisch wurde ein p,p'-Bisphenol A-Gehalt von 91,18 Gew.-% bestimmt.

Zum Vergleich wurde die zweite Teilprobe desselben Reaktionsgemisches ebenfalls bei 120°C/100 mm von Phenol befreit und der Rückstand bei 180°C 30 Minuten lang getempert. Der Gehalt an p,p'-Bisphenol A wurde zu 88,01 Gew.-% bestimmt.

Zum Vergleich wurde außerdem der Rückstand nach dem Entfernen des Phenols ohne zu tempern gaschromatographisch untersucht: Der Gehalt an p,p'-Bisphenol A betrug 89,25 %, der Gehalt an o,p'-Bisphenol A betrug 8,58 %.
A.3) Wie A.2, aber mit 20 g Magnesiumoxid anstelle des Aluminiumoxids.
   p,p'-Bisphenol A-Gehalt nach dem Tempern: 90,73 Gew.-%
   o,p'-Bisphenol A-Gehalt nach dem Tempern: 7,21 Gew.-%
A.4) Wie A.2, aber mit 20 g Calciumoxid anstelle des Aluminiumoxids.
   p,p'-Bisphenol A-Gehalt nach dem Tempern: 90,91 Gew.-%
   o,p'-Bisphenol A-Gehalt nach dem Tempern: 7,33 Gew.-%
A.5) Wie A.2, aber mit 20 g Bariumoxid anstelle des Aluminiumoxids.
   p,p'-Bisphenol A-Gehalt nach dem Tempern: 90,54 Gew.-%
   o,p'-Bisphenol A-Gehalt nach dem Tempern: 7,51 Gew.-%
A.6) Wie A.2, aber mit 20 g Titandioxid anstelle des Aluminiumoxids,
   p,p'-Bisphenol A-Gehalt nach dem Tempern: 90,01 Gew.-%
   o,p'-Bisphenol A-Gehalt nach dem Tempern: 7,63 Gew.-%
A.7) Wie A.2, aber mit 20 g Kieselgel (SiO₂) anstelle des Aluminiumoxids,
   p,p'-Bisphenol A-Gehalt nach dem Tempern: 90,15 Gew.-%
   o,p'-Bisphenol A-Gehalt nach dem Tempern: 7,72 Gew.-%
A.8) Wie A.2, aber mit 20 g Zinkoxid anstelle des Aluminiumoxids,
   p,p'-Bisphenol A-Gehalt nach dem Tempern: 90,84 Gew.-%
   o,p'-Bisphenol A-Gehalt nach dem Tempern: 7,12 Gew.-%
A.9) Wie A.2, aber mit 20 g Tonerde (Tonsil Optimum FF der Firma Südchemie) anstelle des Aluminiuumoixids
   p,p'-Bisphenol A-Gehalt nach dem Tempern: 91,01 Gew.-%
   o,p'-Bisphenol A-Gehalt nach dem Tempern: 7,10 Gew.-%
A.10) Wie A.2, aber mit 20 g Zeolith (Ca-Na-Form, Baylith K 154 der Bayer AG) anstelle des Aluminiumoxids,
   p,p'-Bisphenol A-Gehalt nach dem Tempern: 90,44 Gew.-%
   o,p'-Bisphenol A-Gehalt nach dem Tempern: 7,65 Gew.-%

### B. Aluminiumoxid im Durchflußversuch

Die in A.1 beschriebene Glassäule wurde mit einer zweiten Glassäule derselben Bauart verbunden, die im unteren Bereich mit einer 50 mm dicken Sandschicht und darüber mit 100 g Aluminiumoxid (s. A.2) gefüllt. Durch beide Säulen wurde ein Reaktionsgemisch der unter A.1 beschriebenen Zusammensetzung gepumpt.

Es wurde jeweils eine Probe von 300 ml hinter Säule 1 und hinter Säule 2 entnommen und das Phenol von beiden Proben wie unter A.2 beschrieben entfernt. Danach wurden beide Proben 30 Minuten lang bei 180°C getempert. Der Gehalt an p,p'-Bisphenol A betrug in Probe 1 87,98 Gew.-%, in Probe 2 91,22 Gew.-%.

### C. Betriebsversuch

Ein Teilstrom (20% des Gesamtstroms) des aus den Reaktoren zur Herstellung von Bisphenol A stammenden Produktstroms wurde durch ein Filter und anschließend von oben nach unten durch einen Behälter geleitet, der mit 30 kg Aluminiumoxidgranulat (Typ entsprechend Beispiel A.2 pro 1 000 Liter/Stunde überzuleitenden Teilstrom gefüllt war. Anschließend wurde der Teilstrom durch ein weiteres Filter in die Produktaufarbeitung geführt und mit dem Rest des Gesamtstroms vereinigt. Unmittelbar nach dem Anfahren des Behälters mit Aluminiumoxid beobachtete man eine deutliche Farbverbesserung des Produktteilstroms hinter dem Aluminiumoxidbehälter gegenüber dem Teilstrom vor dem Behälter:

Iodfarbzahl vor dem Aluminiumoxidbehälter: 40; Iodfarbzahl hinter dem Aluminiumoxidbehälter: <10.

Die Verbesserung der Hazen Farbzahl der Bisphenolschmelze, die aus diesem Teilstrom gewonnen wird, zeigte sich aufgrund der Verweilzeiten und Rückvermischungen in den zwischengeschalteten Aufarbeitungsstufen erst nach 7 Tagen in vollem Ausmaß. In dieser Zeit sank die Hazen Farbzahl der Bisphenolschmelze von >10 auf einen Wert <5.

## Patentansprüche

1. Verfahren zur Herstellung thermostabiler Bisphenole, bei dem
a) in im Prinzip bekannter Weise ein Phenol und ein Keton oder Aldehyd katalytisch an einem sauren Ionenaustauscherharz in Gegenwart eines Cokatalysators zu dem entsprechenden Bisphenol umgesetzt werden,
b) das nach a) erzeugte Reaktionsgemisch durch einen Behälter, welcher mit einem anorganischen Oxid gefüllt ist, geleitet wird,
c) aus dem nach b) gewonnenen Reaktionsgemisch in im Prinzip bekannter Weise Bisphenol isoliert wird.

2. Ein Verfahren gemäß Anspruch 1, dadurch gekennzeichnet, daß das anorganische Oxid ein Aluminiumoxid ist.

3. Ein Verfahren gemäß Anspruch 1, dadurch gekennzeichnet, daß das anorganische Oxid Al₂O₃ mit einem Anteil von 0,1 Gew.-% bis 1,0 Gew.-% Natriumoxid und/oder Kaliumoxid ist.

4. Verwendung der gemäß Anspruch 1 hergestellten Bisphenole für die Darstellung von Epoxidharzen, Polyestern, Polyestercarbonaten und Polycarbonaten.
